# EUROPÄISCHE PATENTSCHRIFT

(11) **EP 1 964 928 B1**
(45) Veröffentlichungstag und Bekanntmachung des Hinweises auf die Patenterteilung: **26.01.2011**
(21) Anmeldenummer: 08003602.3
(22) Anmeldetag: 27.02.2008
(51) Int. Cl.: C12Q 1/26, C12Q 1/54, G01N 33/52

(54) **Chinone als Mediatoren für photometrische Teste**
Quinones as mediators for photometric tests
Quinone en tant que médiateurs pour tests photométriques

(30) Priorität: 27.02.2007 EP 07004083
(43) Veröffentlichungstag der Anmeldung: 03.09.2008
(73) Patentinhaber: F.Hoffmann-La Roche AG, 4070 Basel (CH); Roche Diagnostics GmbH, 68305 Mannheim (DE)
(72) Erfinder: Knappe, Wolfgang, 67071 Ludwigshafen (DE)
(74) Vertreter: Weiss, Wolfgang

(56) Entgegenhaltungen:
- EP-A- 1 566 637
- EP-A1- 0 831 327
- WO-A-99/19507
- HILT G ET AL: "An analytical study of the redox behavior of 1,10-phenantroline-5,6-dione, its transition-metal complexes, and its N-monomethylated derivative with regard to their efficiency as mediators of NAD(P)+ regeneration" CHEMISTRY - A EUROPEAN JOURNAL, VCH PUBLISHERS, US, Bd. 3, Nr. 1, 1997, Seiten 79-88, XP002238427 ISSN: 0947-6539

## Beschreibung

Die vorliegende Anmeldung betrifft ein Verfahren zur optischen Detektion eines Analyten in einer Probe, ein zu diesem Zweck geeignetes Nachweisreagenz, Kits sowie Testelemente.

Messsysteme zur biochemischen Analytik stellen wichtige Bestandteile klinisch relevanter Analyseverfahren dar. Hierbei steht die Messung von Analyten im Vordergrund, welche mit Hilfe von Enzymen direkt oder indirekt bestimmt werden. Die Analyten werden hierbei mit Hilfe eines Enzym-Coenzym-Komplexes umgesetzt und anschließend, gegebenenfalls unter Verwendung zusätzlicher Reagenzien, quantifiziert. Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion verändert, z.B. oxidiert bzw. reduziert. Dieser Vorgang kann direkt oder indirekt elektrochemisch oder/und photometrisch erfasst werden. Eine Kalibrierung liefert einen direkten Zusammenhang des Messwerts mit der Konzentration des zu bestimmenden Analyten.

Die aus dem Stand der Technik bekannten Analyseverfahren sind jedoch mit gewissen Nachteilen verbunden. So bedienen sich beispielsweise zahlreiche Teststreifen, welche eine photometrische Detektion eines Analyten gestatten, eines Nachweissystems, welches als Enzym Glucose-Dye-Oxidoreduktase (GlucDOR; EC 1.1.5.2), eine PQQ-abhängige Glucose-Dehydrogenase, verwendet. Während das Substrat im Rahmen der enzymatischen Umsetzung oxidiert wird, erfolgt eine gleichzeitige Reduktion des entsprechenden Coenzyms. Im Falle des Coenzyms PQQ (PQQ: Pyrrolochinolinchinon; 4,5-Dihydro-4,5-dioxo-1 H-pyrrolo[2,3-f]chinolin-2,7,9-tricarbonsäure) erfolgt eine Reduktion zu PQQH₂, welches seiner seits Elektronen auf einen reduzierbaren optischen Indikator übertragen kann. Der Nachteil dieses Systems besteht in der geringen Spezifität von Glucose-Dye-Oxidoreduktase, welche neben Glucose auch andere Saccharide wie beispielsweise Maltose und Galaktose umsetzt und somit aufgrund von Nebenreaktion grobfalsche Ergebnisse liefern kann.

Die NAD⁺-abhängige Glucose-Dehydrogenase (EC 1.1.1.47) stellt ein Enzym dar, welches in Anwesenheit des Coenzyms Nicotinamid-Adenin-Dinukleotid (NAD⁺) die Oxidation von Glucose zu Glucono-δ-lacton katalysiert und eine deutlich höhere Spezifität für Glucose besitzt als Glucose-Dye-Oxidoreduktase. So setzt Glucose-Dehydrogenase im Küvettentest (oder in nasschemischen Tests oder in Lösung) neben Glucose lediglich Xylose und Mannose in 15% bzw. 8% um, während Galaktose und Fructose keine Substrate des Enzyms darstellen (Tietz Textbook of Clinical Chemistry, W. B. Saunders Company, 2. Auflage, 1994, Hrsg. C. A. Burtis, E. R. Ashwood, Seiten 964-965).

Das im Rahmen der Oxidation von Glucose mittels Glucose-Dehydrogenase durch Reduktion von NAD⁺ gebildete NADH besitzt lediglich eine geringe Reaktivität und setzt sich mit reduzierbaren optischen Indikatoren, wie beispielsweise Molybdatophosphorsäure oder 4-Nitrosoanilinen, nicht direkt um. Um dieser Reaktionsträgheit von NADH entgegenzuwirken, werden in der Praxis Mediatoren eingesetzt, welche die Reaktivität des Coenzyms erhöhen. Als Mediatoren sind beispielsweise Diaphorase (EC 1.6.99.2) oder das instabile N-Methylphenazoniummethosuffat bekannt.

EP 0 831 327 A1 betrifft die Verwendung von redoxaktiven Verbindungen zur Herstellung von Nachweisreagenzien für ein Verfahren zur Bestimmung eines Analyten, sowie Reagenzienkits, welche diese redoxaktiven Verbindungen enthalten. Als redoxaktive Verbindungen werden in diesem Zusammenhang Verbindungen mit Benzochinoxalindion-Substruktur beschrieben, welche u.a. auch als Mediatoren bei kolorimetrischen und elektrochemischen Anwendungen eingesetzt werden können.

EP 1 566 637 A1 offenbart Testelemente und Verfahren zum optischen Nachweis von Analyten, wobei die Testelemente einen zumindest teilweise optisch im wesentlichen transparenten Träger sowie einen Einschicht-Reaktionsfilm, enthaltend ein Testreagenz zur Bestimmung des Analyten, mit einer Dicke von ≤ 10 µm umfassen. Das Testreagenz umfasst vorzugsweise mindestens ein Enzym und mindestens ein nach enzymatischer Reaktion direkt oder indirekt nachweisbares Enzymsubstrat.

WO 99/19507 offenbart die Verwendung von Phenanthrolinchinon-Verbindungen als Mediatoren im Rahmen eines amperometrischen Nachweises von Analyten. Hierbei wird eine auf einen Analyten hin zu untersuchende Probe auf einen elektrochemischen Teststreifen, umfassend einen Träger, eine Arbeitselektrode, eine Referenz-/Gegenelektrode sowie leitende Elemente, aufgebracht, der Analyt mittels eines Nicotinamid-abhängigen Enzyms in Gegenwart eines Nicotinamid-Coenzyms und des Mediators oxidiert, und der zur Reoxidation des reduzierten Mediators benötigte Strom, welcher mit der Konzentration des Analyten korreliert, gemessen.

WO 2004/038401 A2 betrifft Biosensoren zur amperometrischen Detektion der Konzentration von Analyten wie beispielsweise Glucose, 3-Hydroxybutyrat und Lactat in einer Probe unter Verwendung elektrochemischer Teststreifen, wie sie in WO 99/19507 beschrieben sind. Als Mediator wird 1,10-Phenanthrolin-5,6-chinon verwendet, welches zum Schutz gegenüber Sauerstoff mit Übergangsmetallionen (z.B. Mangan, Eisen, Osmium, etc.) oder schwereren Erdalkalimetallionen (z.B. Calcium, Barium, etc.) kombiniert wird.

Der Nachteil der in WO 99/19507 und WO 2004/038401 A2 beschriebenen Verfahren liegt in der Verwendung elektrochemischer Testelemente, welche im Rahmen einer qualitativen bzw. quantitativen Detektion des Analyten keine visuelle Überprüfung der Plausibilität von Messergebnissen über Vergleichsfarben gestatten.

Die der Erfindung zugrunde liegende Aufgabe bestand deshalb darin, ein Verfahren zur Detektion von Analyten in einer Probe bereitzustellen, welches eine einfache und kostengünstige Durchführung bei gleichzeitig hoher Selektivität und Messsicherheit gewährleistet.

Diese Aufgabe wurde erfindungsgemäß gelöst mittels eines Verfahrens zur optischen Detektion eines Analyten in einer Probe, umfassend die Schritte:
a) Inkontaktbringen der Probe mit einem Nachweisreagenz, umfassend eine Nicotinamid-abhängige Oxidoreduktase, ein reduzierbares Nicotinamid-Coenzym, einen Mediator, welcher ein Chinon ist, und einen reduzierbaren optischen Indikator oder ein reduzierbares optisches Indikatorsystem,
   wobei der Analyt durch die Nicotinamid-abhängige Oxidoreduktase oxidiert wird, das Nicotinamid-Coenzym reduziert und Elektronen des reduzierten Nicotinamid-Coenzyms durch den Mediator auf den optischen Indikator oder das optische Indikatorsystem übertragen werden, und
b) Bestimmen des Vorhandenseins oder/und der Menge des Analyten in der Probe durch einen optischen Nachweis des Indikators oder des Indikatorsystems,
wobei die Nicotinamid-abhängige Oxidoreduktase Alkohol-Dehydrogenase (EC 1.1.1.1), Glucose-Dehydrogenase (EC 1.1.1.47) oder Glucose-6-phosphat-Dehydrogenase (EC 1.1.1.49) ist, der optische Indikator eine Heteropolysäure ist und der Mediator ein 1,10-Phenanthrolinchinon, ein 1,7-Phenanthrolinchinon, ein 4,7-Phenanthrolinchinon, oder ein N-alkyliertes oder ein N,N'-dialkyliertes Salz hiervon ist.

Das erfindungsgemäße Verfahren dient der optischen Detektion eines Analyten in einer Probe, welche aus einer beliebigen Quelle stammen kann. In einer bevorzugten Ausführungsform stammt die Probe aus einem Körperfluid, umfassend, jedoch nicht beschränkt auf, Vollblut, Plasma, Serum, Lymphflüssigkeit, Gallenflüssigkeit, Cerebrospinalflüssigkeit, Harn, sowie Drüsensekrete wie beispielsweise Speichel oder Schweiß, wobei Vollblut, Plasma und Serum als besonders bevorzugt anzusehen sind. Die zur Durchführung der Analyse benötigte Probenmenge beträgt üblicherweise von etwa 0.01 µl bis etwa 100 µl, bevorzugt von etwa 0.1 µl bis etwa 2 µl.

Der qualtitativ und quantitativ zu bestimmende Analyt ist eine biologische oder chemische Substanz, welche mittels einer Redoxreaktion nachgewiesen werden kann. Vorzugsweise wird der Analyt aus der Gruppe bestehend aus Alkohol und Glucose ausgewählt. In einer besonders bevorzugten Ausführungsform handelt es sich bei dem zu bestimmenden Analyten um Glucose.

Im Rahmen der vorliegenden Erfindung wird die Probe zur Detektion des Analyten mit einem ebenfalls erfindungsgemäßen Nachweisreagenz, welches eine Nicotinamid-abhängige Oxidoreduktase, ein reduzierbares Nicotinamid-Coenzym, einen Mediator, welcher ein Chinon ist, und einen reduzierbaren optischen Indikator oder ein reduzierbares optisches Indikatorsystem umfasst, in Kontakt gebracht, wobei der Analyt durch die Nicotinamid-abhängige Oxidoreduktase oxidiert wird, das Nicotinamid-Coenzym dabei reduziert wird und Elektronen des reduzierten Nicotinamid-Coenzyms durch den Mediator auf den optischen Indikator oder das optische Indikatorsystem übertragen werden.

Die Nicotinamid-abhängige Oxidoreduktase ist eine Alkohol-Dehydrogenase (EC 1.1.1.1), Glucose-Dehydrogenase (EC 1.1.1.47) oder Glucose-6-phosphat-Dehydrogenase (EC 1.1.1.49). Bevorzugt handelt es sich bei der Nicotinamid-abhängigen Oxidoreduktase um Glucose-Dehydrogenase.

Die vorliegende Erfindung sieht vor, dass das reduzierbare Nicotinamid-Coenzym ein beliebiges natürliches oder synthetisches niedermolekulares Molekül ist, welches Nicotinamid als einen Bestandteil enthält, reduzierbar ist und die Fähigkeit besitzt, mit einer Nicotinamid-abhängigen Oxidoreduktase, wie sie vorstehend beschrieben ist, einen Enzym-Coenzym-Komplex auszubilden. Bevorzugt handelt es sich bei dem reduzierbaren Nicotinamid-Coenzym der vorliegenden Erfindung um ein natürlich vorkommendes Nicotinamid-Coenzym, inbesondere um Nicotinamid-Adenin-Dinukleotid (NAD⁺) oder Nicotinamid-Adenin-Dinukleotidphosphat (NADP⁺), aus denen durch Reduktion NADH bzw. NADPH entstehen. Gegebenenfalls können, sofern dies zweckdienlich erscheint, allerdings auch Derivate von NAD⁺ bzw. NADP⁺ eingesetzt werden. Derivate von NAD⁺ bzw. NADP⁺, welche im Rahmen der vorliegenden Erfindung von Nutzen sein können, umfassen unter anderem CarbaNAD-Derivate und sind beispielsweise in WO 98/33936, WO 01/94370, DE 10 2006 035 020.0 und in zwei Publikationen aus dem Jahr 1989 (Slama et al., Biochemistry, 1989, 27, 183-193; Slama et al., Biochemistry, 1989, 28, 7688-7694) beschrieben, auf deren Offenbarung hiermit Bezug genommen wird.

Das Nachweisreagenz, welches im Rahmen des erfindungsgemäßen Verfahrens eingesetzt wird, enthält neben der Nicotinamid-abhängigen Oxidoreduktase und dem reduzierbaren Nicotinamid-Coenzym ferner einen Mediator, welcher ein Chinon ist. Der Mediator ist in der Lage, Elektronen eines reduzierten Nicotinamid-Coenzyms über Redoxreaktionen auf einen optischen Indikator oder ein optisches Indikatorsystem zu übertragen. Im Rahmen dieser Elektronenübertragung wird das Chinon im ersten Schritt zu einem Semichinon bzw. einem Dihydrochinon reduziert, bevor eine Reoxidation der reduzierten Form durch den optischen Indikator oder das optische Indikatorsystem erfolgt.

Erfindungsgemäß wird als Mediator ein 1,10-Phenanthrolinchinon, 1,7-Phenanthrolinchinon, 4,7-Phenanthrolinchinon, oder ein N-alkyliertes oder N,N'-dialkyliertes Salz hiervon eingesetzt. Im Falle N-alkylierter bzw. N,N'-dialkylierter Salze vorstehender Verbindungen kann ein beliebiges Anion als Gegenion des Mediators fungieren, wobei Halogenide, Trifluormethansulfonat oder andere die Löslichkeit erhöhende Anionen als Gegenion bevorzugt sind. In einer besonders bevorzugten Ausführungsform wird als Gegenion ein Halogenid oder Trifluormethansulfonat eingesetzt.

Bevorzugt handelt es sich bei dem Mediator um ein 1,10-Phenanthrolin-5,6-chinon der allgemeinen Formel (I), ein 1,7-Phenanthrolin-5,6-chinon der allgemeinen Formel (II) oder um ein 4,7-Phenanthrolin-5,6-chinon der allgemeinen Formel (III), 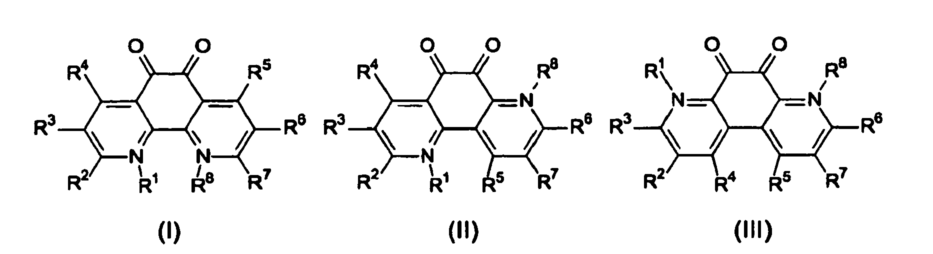 worin
R² bis R⁷ unabhängig voneinander für H, Halogen, OH, O(Alkyl), OCO(Alkyl), S(Alkyl), NH₂, NH(Alkyl), N(Alkyl)₂, [N(Alkyl)₃]⁺, CN, NO₂, COOH, SO₃H, oder für einen linearen oder verzweigten Allylrest, einen Cycloalkylrest, einen Arylrest oder einen Heteroarylrest stehen, welche gegebenenfalls jeweils einfach oder mehrfach substituiert sein können, und
R¹ und R⁸ unabhängig voneinander für ein freies Elektronenpaar, für H oder für einen linearen oder verzweigten Alkylrest, welcher gegebenenfalls einfach oder mehrfach substituiert sein kann, stehen,
oder ein Salz hiervon.

Vorzugsweise steht einer der Reste R¹ und R⁸ oder stehen beide Reste R¹ und R⁸ für einen linearen oder verzweigten Alkylrest, welcher gegebenenfalls einfach oder mehrfach substituiert sein kann, und insbesondere für einen Methylrest, wobei das Gegenion des Mediators wie oben definiert ist.

Der Begriff "Halogen" umfasst Fluor, Chlor, Brom und lod.

Der Begriff "Alkyl" bezeichnet einen linearen oder verzweigten Kohlenwasserstoffrest mit 1-30 Kohlenstoffatomen und einer Bindungsvalenz an einem beliebigen Kohlenstoffatom des Restes. Bevorzugt stellt Alkyl einen Kohlenwasserstoffrest mit 1-12. Kohlenstoffatomen, stärker bevorzugt mit 1-6 Kohlenstoffatomen, dar. Besonders bevorzugt sind Kohlenwasserstoffreste mit 1-4 Kohlenstoffatomen, welche Methyl, Ethyl, n-Propyl, iso-Propyl, n-Butyl, isoButyl, sec-Butyl und tert-Butyl umfassen.

Der Begriff "Cycloalkyl" bezeichnet einen cyclischen Kohlenwasserstoffrest mit 3-20 Kohlenstoffatomen und einer Bindungsvalenz an einem beliebigen Kohlenstoffatom des Ringes. Bevorzugt stellt Cycloalkyl einen cyclischen Kohlenwasserstoffrest mit 3-12 Kohlenstoffatomen, stärker bevorzugt mit 3-8 Kohlenstoffatomen, dar. Besonders bevorzugte cyclische Kohlenwasserstoffreste umfassen Cyclopropyl, Cyclobutyl, Cyclopentyl, Cyclohexyl, Cycloheptyl und Cyclooctyl.

Der Begriff "Aryl" bezeichnet ein aromatisches Ringsystem mit 3-20 Ringatomen, stärker bevorzugt mit 6-14 Ringatomen, welches neben Kohlenstoff ausschließlich Wasserstoff enthält und eine Bindungsvalenz an einem beliebigen Kohlenstoffatom des Ringes aufweist. Besonders bevorzugte Beispiele für Aryle im Sinne der vorliegenden Erfindung umfassen Benzol, Naphthalin, Anthracen und Phenanthren.

Der Begriff "Heteroaryl" bezeichnet ein aromatisches Ringsystem mit 3-20 Ringatomen, stärker bevorzugt mit 5-14 Ringatomen, welches neben Kohlenstoff und Wasserstoff mindestens ein Heteroatom enthält und eine Bindungsvalenz an einem beliebigen Kohlenstoffatom oder an einem beliebigen Stickstoffatom des Ringes aufweist. Beispiele für Heteroaryle mit 5 Ringatomen umfassen Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl- und Thiadiazolyl. Heteroaryle mit 6 Ringatomen umfassen beispielsweise Pyridyl, Pyridazinyl, Pyrimidinyl, Pyrazinyl und Triazinyl. Heteroaryle mit 5 bzw. 6 Ringatomen besitzen bevorzugt 1-4 Stickstoffatome oder/und 1-2 Sauerstoffatome oder/und 1-2 Schwefelatome, welche in allen Unterkombinationen im Ringsystem auftreten können, solange sie die für das jeweilige Heteroatom festgelegte Anzahl und in der Summe die Höchstzahl von vier Heteroatomen nicht überschreiten.

Der Ausdruck "gegebenenfalls einfach oder mehrfach substituiert" bedeutet, dass der jeweilige Rest entweder unsubstituiert oder aber einfach oder mehrfach substituiert sein kann, wobei als Substituenten insbesondere Halogen, OH, O(Alkyl), OCO(Alkyl), S(Alkyl), primäre, sekundäre, tertiäre und quartäre Aminogruppen, CN, NO₂ oder/und Säuregruppen, wie beispielsweise COOH und SO₃H in Frage kommen. Besonders bevorzugt handelt es sich bei jenen Substituenten um Gruppen, welche die Löslichkeit des Mediators in der zu untersuchenden Probe erhöhen, wie beispielsweise quartäre Aminogruppen, COOH und SO₃H.

In der am stärksten bevorzugten Ausführungsform wird zur Durchführung des erfindungsgemäßen Verfahrens N-Methyl-1,10-Phenanthrolinium-5,6-chinon, N,N'-Dimethyl-1,10-Phenanthrolinium-5,6-chinon, N-Methyl-1,7-Phenanthrolinium-5,6-chinon, N,N'-Dimethyl-1,7-Phenanthrolinium-5,6-chinon, N-Methyl-4,7-Phenanthrolinium-5,6-chinon oder N,N'-Dimethyl-4,7-Phenanthrolinium-5,6-chinon als Mediator verwendet, wobei das Gegenion des Mediators wie oben definiert ist.

Als optischer Indikator oder als optisches Indikatorsystem wird eine Substanz verwendet, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe durch optischen Nachweis kann mit dem bloßen Auge oder/und mittels einer Detektionsvorrichtung unter Verwendung eines dem Fachmann geeignet erscheinenden photometrischen Verfahrens erfolgen, wobei eine Auslesung mit dem bloßen Auge als bevorzugt anzusehen ist. Erfindungsgemäß wird als optischer Indikator eine Heteropolysäure, und insbesondere Molybdatophosphorsäure, verwendet, die zum entsprechenden Heteropolyblau reduziert wird.

In einer weiteren bevorzugten Ausführungsform wird das erfindungsgemäße Verfahren auf einem Träger durchgeführt, welcher einen Applikationsbereich zum Aufbringen der Probe, einen Reaktionsbereich zum Umsetzen des Analyten mit dem Nachweisreagenz, einen Detektionsbereich zum Bestimmen des Vorhandenseins oder/und der Menge des Analyten in der Probe durch einen optischen Nachweis des Indikators oder des Indikatorsystems, und gegebenenfalls einen Abfallbereich umfasst. Der Träger kann aus einem einzelnen kapillaraktiven Material bestehen, oder kann alternativ aus mehreren kapillaraktiven Materialien, welche gleich oder verschieden sind, zusammengesetzt sein. Diese Materialien stehen in engem Kontakt miteinander, so dass auf diese Weise ein dem Transport von Flüssigkeit dienender Weg gebildet wird, über welchen sich eine flüssige Probe vom Applikationsbereich über den Reaktionsbereich zum Detektionsbereich und gegebenenfalls zum Abfallbereich fortbewegt. In einer bevorzugten Ausführungsform handelt es sich bei dem Träger um ein Testelement, wie beispielsweise einen Teststreifen oder einen Biosensor.

Testelemente, welche im Rahmen der vorliegenden Erfindung verwendet werden können, sind unter anderem in U.S. 5,271,895, U.S. 6,207,000, U.S.6,540,890, U.S. 6,755,949, U.S. 7,008,799, U.S.7,025,836, U.S. 7,067,320, U.S. 2003/0031592 A1 und U.S. 2006/0003397 A1 beschrieben, auf deren Offenbarung hiermit Bezug genommen wird.

Vorzugsweise wird die zu untersuchende Probe direkt auf den Applikationsbereich des Trägers aufgebracht, indem beispielsweise der Applikationsbereich des Trägers in die Probe eingetaucht oder die Probe auf den Applikationsbereich des Trägers aufgetropft wird. Alternativ besteht die Möglichkeit, die Probe zunächst mittels eines trockenen oder feuchten Transferelements aufzunehmen, von welchem die Probe anschließend, gegebenenfalls nach Befeuchtung, auf den Applikationsbereich des Trägers aufgebracht wird. Üblicherweise handelt es sich bei dem Transferelement um eine sterile Vorrichtung, welche natürliche oder/und synthetische Materialien umfassen kann. Geeignete Transferelemente sind beispielsweise in DE 44 39 429 und DE 196 22 503 beschrieben, auf deren Offenbarung hiermit Bezug genommen wird.

Der Reaktionsbereich umfasst das erfindungsgemäße Nachweisreagenz und dient der Umsetzung des Analyten. Im Rahmen dieser Umsetzung wird der Analyt durch die Nicotinamid-abhängige Oxidoreduktase oxidiert, während eine gleichzeitige Reduktion des Nicotinamid-Coenzyms stattfindet. Die Elektronen des reduzierten Nicotinamid-Coenzyms werden mittels des Mediators in der Folge auf den optischen Indikator oder das optische Indikatorsystem übertragen, welcher/welches eine Änderung seiner optischen Eigenschaften erfährt. Die Änderung optischer Eigenschaften des optischen Indikators oder des optischen Indikatorsystems wird im Detektionsbereich des Testelements nachgewiesen.

In einer besonders bevorzugten Ausführungsform gestattet das erfindungsgemäße Verfahren eine schnelle Bestimmung des Vorhandenseins oder/und der Menge des Analyten in der zu untersuchenden Probe. Der Ausdruck "schnelle Bestimmung", wie er im Rahmen der vorliegenden Anmeldung verwendet wird, bedeutet, dass die Bestimmung des Vorhandenseins oder/und der Menge des Analyten innerhalb eines Zeitraums von 1 bis 30 Sekunden nach Inkontaktbringen der zu untersuchenden Probe mit dem Nachweisreagenz erfolgt, wobei ein Zeitraum von 2 bis 15 Sekunden als bevorzugt angesehen wird.

Um derart kurze Reaktionszeiten zu gewährleisten, ist es bevorzugt, dass sich der Mediator in der zu untersuchenden Probe schnell auflöst, d. h. beispielsweise innerhalb eines Zeitraumes von wenigen Sekunden nach Inkontaktbringen der Probe mit dem Nachweisreagenz. Eine schnelle Auflösung des Mediators kann beispielsweise durch Einführung geeigneter, die Löslichkeit erhöhender Substituenten in das Mediatormolekül, durch Einkapselung des Mediators in Mizellen, durch eine sehr feine, nahezu amorphe Verteilung des Mediators in einem Testelement, oder/und bei Salzen durch Wahl eines geeigneten Gegenions erzielt werden.

In einem weiteren Aspekt betrifft die vorliegende Erfindung einen Kit zur optischen Detektion eines Analyten in einer Probe, welcher das erfindungsgemäße Nachweisreagenz und ein Testelement umfasst. Das Testelement umfasst einen Applikationsbereich zum Aufbringen der Probe, einen Reaktionsbereich zum Umsetzen des Analyten mit dem Nachweisreagenz, einen Detektionsbereich zum Bestimmen des Vorhandenseins oder/und der Menge des Analyten in der Probe durch einen optischen Nachweis des Indikators oder des Indikatorsystems, und gegebenenfalls einen Abfallbereich, wobei hinsichtlich möglicher Ausgestaltungen des Testelements auf die Ausführungen im Rahmen der Beschreibung des erfindungsgemäßen Verfahrens verwiesen wird.

In noch einem weiteren Aspekt betrifft die vorliegende Erfindung ein Testelement zur optischen Detektion eines Analyten in einer Probe. Das Testelement umfasst einen Applikationsbereich zum Aufbringen der Probe, einen Reaktionsbereich, welcher das erfindungsgemäße Nachweisreagenz, umfassend eine Nicotinamid-abhängige Oxidoreduktase, ein reduzierbares Nicotinamid-Coenzym, einen Mediator, welcher ein Chinon ist, und einen reduzierbaren optischen Indikator oder ein reduzierbares optisches Indikatorsystem, enthält, zum Umsetzen des Analyten mit dem Nachweisreagenz, einen Detektionsbereich zum Bestimmen des Vorhandenseins oder/und der Menge des Analyten in der Probe durch einen optischen Nachweis des Indikators oder des Indikatorsystems, und gegebenenfalls einen Abfallbereich.

Das erfindungsgemäße Testelement kann beipielsweise zur Bestimmung von Analyten aus der Gruppe bestehend aus Alkohol und Glucose verwendet werden. Bevorzugt ist im Rahmen der vorliegenden Erfindung ein Testelement, welches der Bestimmung von Glucose in Vollblut, Plasma oder Serum dient und ein Nachweisreagenz umfasst, das Glucose-Dehydrogenase als Nicotinamid-abhängige Oxidoreduktase und NAD(P)⁺ als Nicotinamid-Coenzym enthält.

Die Erfindung soll durch die nachfolgenden Figuren und Beispiele näher erläutert werden.

### Abbildung

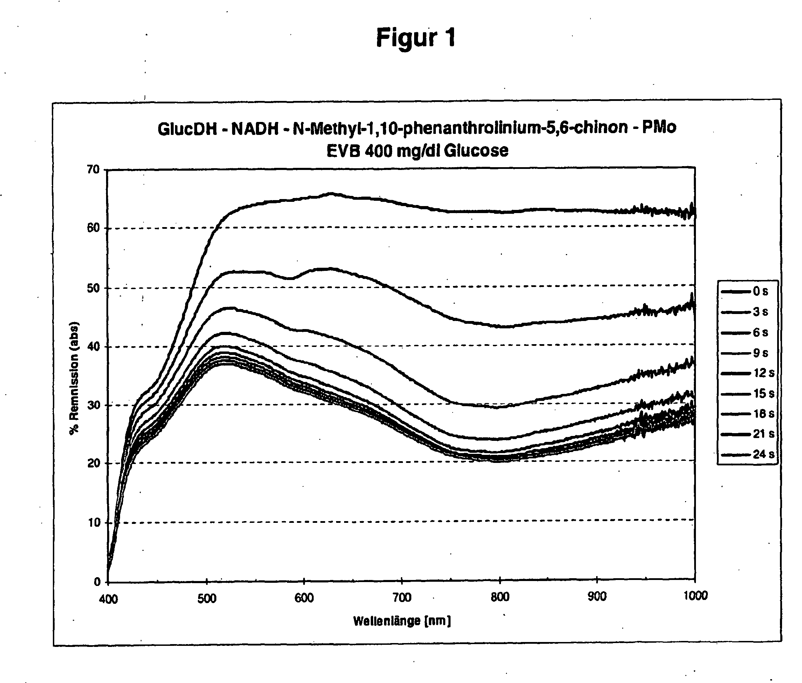

Figur 1 zeigt die Remission eines erfindungsgemäßen Testelements mit N-Methyl-1,10-phenanthroliniurn-5,6-chinon als Mediator in Abhängigkeit von der Wellenlänge vor und nach Inkontaktbringen mit einer 400 mg/dl an Glucose enthaltenden Probe von 10 µl EDTA-Venenblut. Die Spektren belegen eine schnelle Reaktion, welche nach 12 s weitgehend beendet ist.

Die Spektren wurden in einem Abstand von jeweils 3 s mittels eines "TIDAS 16"-Remissionspektrometers aufgenommen, welches aus einer Wolfram-Halogenlichtquelle CLH, einem Remissionsmesskopf und einem Diodenarray-Simultanspektrometer MCS (jeweils Fa. Zeiss) bestand, die ihrerseits mit Lichtleitern verbunden waren.

### Beispiel

Zur Herstellung erfindungsgemäßer Testelemente wurde auf eine bandförmige, 50 mm breite Titandioxid-haltige Polyestertragschicht parallel im Abstand von 18,6 mm (gemessen zur linken Kante des Klebebandes) zu seiner linken Kante zunächst ein 5 mm breites doppelseitiges Klebeband (Polyesterträger und Synthesekautschukkleber) aufgebracht. Aus diesem Verbund wurden mit einem Abstand von 6 mm jeweils zwei Löcher, ein Positionierungsloch und ein Inspektions- und Messloch, ausgestanzt, deren Mittelpunkte auf einer senkrecht zur Längsachse des Trägerstreifens liegenden Gerade lagen. Das erste Loch, das Positionierungsloch, war kreisrund und wies einen Durchmesser von 2,6 mm auf, wobei der Abstand des Lochmittelpunkts von der linken Kante des Trägerstreifens 4 mm betrug. Das zweite Loch war ebenfalls rund mit einem Durchmesser von 4 mm. Der Mittelpunktsabstand des zweiten Lochs von der linken Kante des Trägerstreifens betrug 21 mm. Anschließend wurde das Schutzpapier des doppelseitigen Klebebandes abgezogen.

Zur Herstellung einer aus 2 Filmschichten aufgebauten Nachweisschicht wurden in einem Becherglas die nachfolgenden Komponenten als Reinsubstanzen oder in Form von Stammlösungen in folgender Zusammensetzung zusammengegeben und durch Rühren vermischt:

| | |
|---|---|
| Wasser: | 60,5 g |
| Xanthan gum: | 0,34 g |
| 0,1 M Phosphatpuffer pH 6,5: | 20,0 g |
| Tetraethylammoniumchlorid: | 0,14 g |
| N-Octanoyl-N-methyl-glucamid: | 0,17 g |
| Natrium-N-methyl-N-oleoyl-taurat: | 0,03 g |
| Polyvinylpyrrolidon (MG 25.000): | 0,87 g |
| Transpafill (Natrium-aluminiumsilikat): | 5,45 g |
| Polyvinylpropionat-Dispersion (50 Gew.-% in Wasser): N-Methyl-1,10-phenantrholinium-5,6-chinon- | 4,88 g |
| trifiluormethylsulfonat: | 0,11 g |
| Natriumchlorid: | 0,98 g |
| NAD: | 0,99 g |
| 2,18-Phosphomolybdänsäure-hexanatriumsalz: | 0,64 g |
| Glucose-Dehydrogenase: | 327 KU (1,35 g) |
| Kaliumhexacyanoferrat(III): | 0,02 g |
| 1-Hexanol: | 0,17 g |
| 1-Methoxy-2-propanol: | 4,30 g |

Die Gesamtmasse wurde mit NaOH auf einen pH-Wert von 6,7 eingestellt und anschließend mit einem Flächengewicht von 89 g/qm auf eine 125 µm dicke Polycarbonatfolie aufgebracht und getrocknet.

Im Anschluss wurden in einem Becherglas die nachfolgenden Komponenten als Reinsubstanzen oder in Form von Stammlösungen in folgender Zusammensetzung zusammengegeben und durch Rühren vermischt:

| | |
|---|---|
| Wasser: | 65,0 g |
| Gantrez (Methylvinylether-Maleinsäure-Copolymer): | 1,36 g |
| NaOH: | 0,30 g |
| 0,1 M Phosphatpuffer pH 6,5: | 4,41 g |
| Tetraethylammoniumchlorid: | 0,34 g |
| N-Octanoyl-N-methyl-glucamid: | 0,34 g |
| Natrium-N-methyl-N-oleoyl-taurat: | 0,03 g |
| Polyvinylpyrrolidon (MG 25.000): | 1,86 g |
| Titandioxid E 171: | 14,37 g |
| Fällungskieselsäure 320: | 1,96 g |
| Polyvinylpropionat-Dispersion (50 Gew.-% in Wasser): N-Methyl-1,10-phenanthrolinium-5,6-chinon- | 5,77 g |
| trifluormethylsulfonat: | 0,38 g |
| 2,18-Phosphomolybdänsäure-hexanatriumsalz: | 1,11 g |
| Kaliumhexacyanoferrat(III): | 0,01 g |
| 1-Hexanol: | 0,16 g |
| 1-Methoxy-2-propanol: | 4,26 g |

Die Gesamtmasse wurde mit NaOH auf einen pH-Wert von ca. 6,7 eingestellt und als zweite Schicht mit einem Flächengewicht von 104 g/qm auf die vorstehend beschriebene, einfach beschichtete Polycarbonatfolie aufgebracht und getrocknet.

Ein 5 mm breiter Streifen der auf diese Weise hergestellten Nachweisschicht wurde mit der Folienseite auf das auf der Tragschicht befindliche, gestanzte doppelseitige Klebeband passgenau aufgeklebt. Direkt an die Nachweisschicht angrenzend wurden auf beiden Seiten doppelseitige Klebebänder als Abstandshalter auf die Trägerfolie aufgeklebt, wobei in vorliegendem Fall ein Abstandshalter 6 mm und der zweite Abstandshalter 9 mm breit war. Anschließend wurde die Schutzfolie der beiden doppelseitigen Klebebänder abgezogen.

Auf diesen Verbund wurde ein 20 mm breiter Streifen eines Spreitvlieses, wie er beispielsweise in EP 0 995 994 A2, auf dessen Offenbarung hiermit Bezug genommen wird, beschrieben ist, aufgelegt und durch Anpressen verklebt. Im Anschluss wurden zwei einseitige Klebebänder als Abdeckungen so auf das Spreitvlies aufgeklebt, dass die Abstandshalter ganz abgedeckt waren und wenigstens noch eine geringfügige Überlappung mit dem Reaktivbezirk vorlag. Die Bandware wurde in 6 mm breite Testelemente geschnitten, wobei das Messloch mittig im Testelement lag.

## Patentansprüche

1. Verfahren zur optischen Detektion eines Analyten in einer Probe, umfassend die Schritte:
(a) Inkontaktbringen der Probe mit einem Nachweisreagenz, umfassend eine Nicotinamid-abhängige Oxidoreduktase, ein reduzierbares Nicotinamid-Coenzym, einen Mediator, welcher ein Chinon ist, und einen reduzierbaren optischen Indikator oder ein reduzierbares optisches Indikatorsystem,
wobei der Analyt durch die Nicotinamid-abhängige Oxidoreduktase oxidiert wird, das Nicotinamid-Coenzym reduziert und Elektronen des reduzierten Nicotinamid-Coenzyms durch den Mediator auf den optischen Indikator oder das optische Indikatorsystem übertragen werden, und
(b) Bestimmen des Vorhandenseins oder/und der Menge des Analyten in der Probe durch einen optischen Nachweis des Indikators oder des Indikatorsystems,
wobei die Nicotinamid-abhängige Oxidoreduktase Alkohol-Dehydrogenase (EC 1.1.1.1), Glucose-Dehydrogenase (EC 1.1.1.47) oder Glucose-6-phosphat-Dehydrogenase (EC 1.1.1.49) ist, der optische Indikator eine Heteropolysäure ist und der Mediator ein 1,10-Phenanthrolinchinon, ein 1,7-Phenanthrolinchinon, ein 4,7-Phenanthrolinchinon, oder ein N-alkyliertes oder ein N,N'-dialkyliertes Salz hiervon ist.

2. Verfahren nach Anspruch 1,
**dadurch gekennzeichnet,**
**dass** die Probe ein Körperfluid ist.

3. Verfahren nach Anspruch 1 oder 2,
**dadurch gekennzeichnet,**
**dass** das Körperfluid Vollblut, Plasma oder Serum ist.

4. Verfahren nach einem der Ansprüche 1 bis 3,
**dadurch gekennzeichnet,**
**dass** der Analyt Alkohol oder Glucose ist.

5. Verfahren nach einem der Ansprüche 1 bis 4,
**dadurch gekennzeichnet,**
**dass** die Nicotinamid-abhängige Oxidoreduktase Glucose-Dehydrogenase (EC 1.1.1.47) ist.

6. Verfahren nach einem der Ansprüche 1 bis 5,
**dadurch gekennzeichnet,**
**dass** das Nicotinamid-Coenzym NAD(P)⁺ ist.

7. Verfahren nach einem der Ansprüche 1 bis 6,
**dadurch gekennzeichnet,**
**dass** der Mediator ein 1,10-Phenanthrolin-5,6-chinon der allgemeinen Formel (I), ein 1,7-Phenanthrolin-5,6-chinon der allgemeinen Formel (II) oder ein 4,7-Phenanthrolin-5,6-chinon der allgemeinen Formel (III) ist, 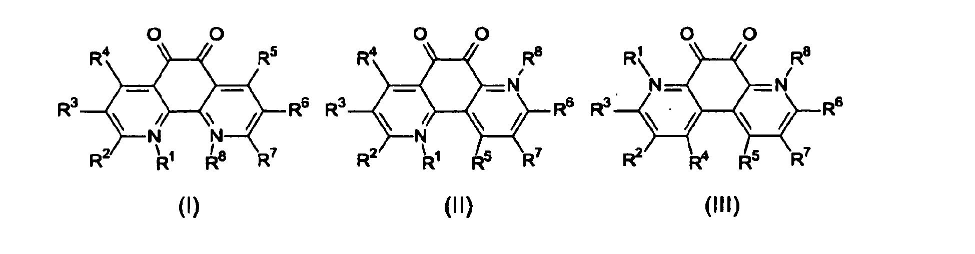 worin
R² bis R⁷ unabhängig voneinander für H, Halogen, OH, O(Alkyl), OCO(Alkyl), S(Alkyl), NH₂, NH(Alkyl), N(Alkyl)₂, [N(Alkyl)₃]⁺, CN, NO₂, COOH, SO₃H, oder für einen linearen oder verzweigten Alkylrest, einen Cycloalkylrest, einen Arylrest oder einen Heteroarylrest stehen, welche gegebenenfalls jeweils einfach oder mehrfach substituiert sein können, und
R¹ und R⁸ unabhängig voneinander für ein freies Elektronenpaar, für H oder für einen linearen oder verzweigten Alkylrest, welcher gegebenenfalls einfach oder mehrfach substituiert sein kann, stehen,
oder ein Salz hiervon.

8. Verfahren nach einem der Ansprüche 1 bis 7,
**dadurch gekennzeichnet, dass** der Mediator N-Methyl-1,10-Phenanthrolinium-5,6-chinon, N,N'-Dimethyl-1,10-Phenanthrolinium-5,6-chinon, N-Methyl-1,7-Phenanthrolinium-5,6-chinon, N,N'-Dimethyl-1,7-Phenanthrolinium-5,6-chinon, N-Methyl-4,7-Phenanthrolinium-5,6-chinon oder N,N'-Dimethyl-4,7-Phenanthrolinium-5,6-chinon ist.

9. Verfahren nach einem der Ansprüche 1 bis 8,
**dadurch gekennzeichnet,**
**dass** der optische Indikator Molybdatophosphorsäure ist.

10. Verfahren nach einem der Ansprüche 1 bis 9,
**dadurch gekennzeichnet,**
**dass** das Verfahren auf einem Träger durchgeführt wird, wobei der Träger umfasst:
(a) einen Applikationsbereich zum Aufbringen der Probe,
(b) einen Reaktionsbereich zum Umsetzen des Analyten mit dem Nachweisreagenz,
(c) einen Detektionsbereich zum Bestimmen des Vorhandenseins oder/und der Menge des Analyten in der Probe durch einen optischen Nachweis des Indikators oder des Indikatorsystems, und
(d) gegebenenfalls einen Abfallbereich.

11. Verfahren nach Anspruch 10,
**dadurch gekennzeichnet,**
**dass** die Probe direkt auf den Applikationsbereich aufgebracht wird.

12. Verfahren nach Anspruch 10,
**dadurch gekennzeichnet,**
**dass** die Probe mittels eines Transferelements aufgenommen und anschließend, gegebenenfalls nach Befeuchtung, auf den Applikationsbereich aufgebracht wird.

13. Verfahren nach einem der Ansprüche 10 bis 12,
**dadurch gekennzeichnet,**
**dass** der Träger ein Testelement ist.

14. Nachweisreagenz zur optischen Detektion eines Analyten in einer Probe, umfassend
(a) eine Nicotinamid-abhängige Oxidoreduktase,
(b) ein reduzierbares Nicotinamid-Coenzym,
(c) einen Mediator, welcher ein Chinon ist, und
(d) einen reduzierbaren optischen Indikator oder ein reduzierbares optisches Indikatorsystem,
wobei die Nicotinamid-abhängige Oxidoreduktase Alkohol-Dehydrogenase (EC 1.1.1.1), Glucose-Dehydrogenase (EC 1.1.1.47) oder Glucose-6-phosphat-Dehydrogenase (EC 1.1.1.49) ist, der optische Indikator eine Heteropolysäure ist und der Mediator ein 1,10-Phenanthrolinchinon, ein 1,7-Phenanthrolinchinon, ein 4,7-Phenanthrolinchinon, oder ein N-alkyliertes oder ein N,N'-dialkyliertes Salz hiervon ist.

15. Verwendung eines Nachweisreagenzes nach Anspruch 14 in einem Testelement.

16. Kit, umfassend ein Nachweisreagenz nach Anspruch 14 und ein Testelement, zur optischen Detektion eines Analyten in einer Probe, wobei das Testelement umfasst,
(a) einen Applikationsbereich zum Aufbringen der Probe,
(b) einen Reaktionsbereich zum Umsetzen des Analyten mit dem Nachweisreagenz,
(c) einen Detektionsbereich zum Bestimmen des Vorhandenseins oder/und der Menge des Analyten in der Probe durch einen optischen Nachweis des Indikators oder des Indikatorsystems, und
(d) gegebenenfalls einen Abfallbereich.

17. Testelement zur optischen Bestimmung eines Analyten in einer Probe, umfassend
(a) einen Applikationsbereich zum Aufbringen der Probe,
(b) einen Reaktionsbereich, welcher ein Nachweisreagenz, umfassend eine Nicotinamid-abhängige Oxidoreduktase, ein reduzierbares Nicotinamid-Coenzym, einen Mediator, welcher ein Chinon ist, und einen reduzierbaren optischen Indikator oder ein reduzierbares optisches Indikatorsystem, enthält, zum Umsetzen des Analyten mit dem Nachweisreagenz,
(c) einen Detektionsbereich zum Bestimmen des Vorhandenseins oder/und der Menge des Analyten in der Probe durch einen optischen Nachweis des Indikators oder des Indikatorsystems, und
(d) gegebenenfalls einen Abfallbereich,
wobei die Nicotinamid-abhängige Oxidoreduktase Alkohol-Dehydrogenase (EC 1.1.1.1), Glucose-Dehydrogenase (EC 1.1.1.47) oder Glucose-6-phosphat-Dehydrogenase (EC 1.1.1.49) ist, der optische Indikator eine Heteropolysäure ist und der Mediator ein 1,10-Phenanthrolinchinon, ein 1,7-Phenanthrolinchinon, ein 4,7-Phenanthrolinchinon, oder ein N-alkyliertes oder ein N,N'-dialkyliertes Salz hiervon ist.

## Claims

1. Method for the optical detection of an analyte in a sample, comprising the steps:
(a) contacting the sample with a detection reagent comprising a nicotinamide-dependent oxidoreductase, a reducible nicotinamide coenzyme, a mediator which is a quinone, and a reducible optical indicator or a reducible optical indicator system,
wherein the analyte is oxidized by the nicotinamide-dependent oxidoreductase, the nicotinamide coenzyme is reduced, and electrons of the reduced nicotinamide coenzyme are transferred by the mediator to the optical indicator or to the optical indicator system, and
(b) determining the presence or/and the amount of the analyte in the sample by optically detecting the indicator or the indicator system,
wherein the nicotinamide-dependent oxidoreductase is alcohol dehydrogenase (EC 1.1.1.1), glucose dehydrogenase (EC 1.1.1.47) or glucose-6-phosphate dehydrogenase (EC 1.1.1.49), wherein the optical indicator is a heteropoly acid, and wherein the mediator is a 1,10-phenanthrolinequinone, a 1,7-phenanthrolinequinone, a 4,7-phenanthrolinequinone, or an N-alkylated or N,N'-dialkylated salt thereof.

2. The method according to claim 1,
**characterized in that**
the sample is a body fluid.

3. The method according to claim 1 or 2,
**characterized in that**
the body fluid is whole blood, plasma or serum.

4. The method according to any one of claims 1 to 3,
**characterized in that**
the analyte is alcohol or glucose.

5. The method according to any one of claims 1 to 4,
**characterized in that**
the nicotinamide-dependent oxidoreductase is glucose dehydrogenase (EC 1.1.1.47).

6. The method according to any one of claims 1 to 5,
**characterized in that**
the nicotinamide coenzyme is NAD(P)⁺.

7. The method according to any one of claims 1 to 6,
**characterized in that**
the mediator is a 1,10-phenanthroline-5,6-quinone of the general formula (I), a 1,7-phenanthroline-5,6-quinone of the general formula (II) or a 4,7-phenanthroline-5,6-quinone of the general formula (III) 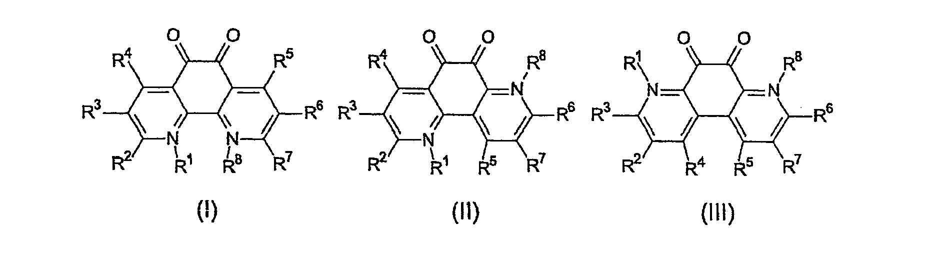 in which
R² to R⁷ are independently of one another H, halogen, OH, O(alkyl), OCO(alkyl), S(alkyl), NH₂, NH(alkyl), N(alkyl)₂, [N(alkyl)₃]⁺, CN, NO₂, COOH, SO₃H, or are a linear or branched alkyl radical, a cycloalkyl radical, an aryl radical or a heteroaryl radical, which may in each case optionally be substituted one or more times, and
R¹ and R⁸ are independently of one another a free electron pair, are H or are a linear or branched alkyl radical which may optionally be substituted one or more times,
or a salt thereof.

8. The method according to any one of claims 1 to 7,
**characterized in that**
the mediator is N-methyl-1,10-phenanthrolinium-5,6-quinone, N,N'-dimethyl-1,10-phenanthrolinium-5,6-quinone, N-methyl-1,7-phenanthrolinium-5,6-quinone, N,N'-dimethyl-1,7-phenanthrolinium-5,6-quinone, N-methyl-4,7-phenanthrolinium-5,6-quinone or N,N'-dimethyl-4,7-phenanthrolinium-5,6-quinone.

9. The method according to any one of claims 1 to 8,
**characterized in that**
the optical indicator is phosphomolybdic acid.

10. The method according to any one of claims 1 to 9,
**characterized in that**
the method is carried out on a carrier, wherein the carrier comprises:
(a) an application zone for applying the sample,
(b) a reaction zone for reacting the analyte with the detection reagent,
(c) a detection zone for determining the presence or/and the amount of the analyte in the sample by optically detecting the indicator or the indicator system, and
(d) optionally a waste zone.

11. The method according to claim 10,
**characterized in that**
the sample is applied directly to the application zone.

12. The method according to claim 10,
**characterized in that**
the sample is taken up by means of a transfer element and then, where appropriate after moistening, applied to the application zone.

13. The method according to any one of claims 10 to 12,
**characterized in that**
the carrier is a test element.

14. Detection reagent for the optical detection of an analyte in a sample, comprising
(a) a nicotinamide-dependent oxidoreductase,
(b) a reducible nicotinamide coenzyme,
(c) a mediator which is a quinone, and
(d) a reducible optical indicator or a reducible optical indicator system, wherein the nicotinamide-dependent oxidoreductase is alcohol dehydrogenase (EC 1.1.1.1), glucose dehydrogenase (EC 1.1.1.47) or glucose-6-phosphate dehydrogenase (EC 1.1.1.49), wherein the optical indicator is a heteropoly acid, and wherein the mediator is a 1,10-phenanthrolinequinone, a 1,7-phenanthrolinequinone, a 4,7-phenanthrolinequinone, or an N-alkylated or N,N'-dialkylated salt thereof.

15. Use of a detection reagent according to claim 14 in a test element.

16. Kit comprising a detection reagent according to claim 14 and a test element for the optical detection of an analyte in a sample, wherein the test element comprises
(a) an application zone for applying the sample,
(b) a reaction zone for reacting the analyte with the detection reagent,
(c) a detection zone for determining the presence or/and the amount of the analyte in the sample by optically detecting the indicator or the indicator system, and
(d) optionally a waste zone.

17. Test element for the optical detection of an analyte in a sample, comprising
(a) an application zone for applying the sample,
(b) a reaction zone which comprises a detection reagent comprising a nicotinamide-dependent oxidoreductase, a reducible nicotinamide coenzyme, a mediator which is a quinone, and a reducible optical indicator or a reducible optical indicator system, for reacting the analyte with the detection reagent,
(c) a detection zone for determining the presence or/and the amount of the analyte in the sample by optically detecting the indicator or the indicator system, and
(d) optionally a waste zone,
wherein the nicotinamide-dependent oxidoreductase is alcohol dehydrogenase (EC 1.1.1.1), glucose dehydrogenase (EC 1.1.1.47) or glucose-6-phosphate dehydrogenase (EC 1.1.1.49), wherein the optical indicator is a heteropoly acid, and wherein the mediator is a 1,10-phenanthrolinequinone, a 1,7-phenanthrolinequinone, a 4,7-phenanthrolinequinone, or an N-alkylated or N,N'-dialkylated salt thereof.

## Revendications

1. Procédé de détection optique d'un analyte dans un échantillon, comprenant les étapes consistant à :
(a) mettre en contact l'échantillon avec un réactif de détection, comprenant une oxydoréductase dépendante du nicotinamide, une co-enzyme de nicotinamide réductible, un médiateur qui est une quinone et un indicateur optique réductible ou un système d'indicateur optique réductible,
dans lequel l'analyte est oxydé par l'oxydoréductase dépendante du nicotinamide, la co-enzyme de nicotinamide est réduite et les électrons de la co-enzyme de nicotinamide réduite sont transmis par le médiateur à l'indicateur optique ou au système d'indicateur optique, et
(b) déterminer la présence ou/et la quantité d'analytes dans l'échantillon par une détection optique de l'indicateur ou du système d'indicateur,
dans lequel l'oxydoréductase dépendante du nicotinamide est l'alcool-déshydrogénase (EC 1.1.1.1), la glucose déshydrogénase (EC 1.1.1.47) ou la glucose-6-phosphate-déshydrogénase (EC 1.1.1.49), l'indicateur optique est un hétéropolyacide et le médiateur est une 1,10-phénanthroline-quinone, une 1,7-phénanthroline-quinone, une 4,7-phénanthroline-quinone ou un sel N-alkylé ou N,N'-dialkylé de celles-ci.

2. Procédé selon la revendication 1,
**caractérisé en ce**
**que** l'échantillon est un fluide corporel.

3. Procédé selon la revendication 1 ou 2,
**caractérisé en ce**
**que** le fluide corporel est du sang total, du plasma ou du sérum.

4. Procédé selon l'une des revendications 1 à 3,
**caractérisé en ce**
**que** l'analyte est un alcool ou du glucose.

5. Procédé selon l'une des revendications 1 à 4,
**caractérisé en ce**
**que** l'oxydoréductase dépendante du nicotinamide est la glucose-déshydrogénase (EC 1.1.1.47).

6. Procédé selon l'une des revendications 1 à 5,
**caractérisé en ce**
**que** la co-enzyme de nicotinamide est la NAD(P)⁺.

7. Procédé selon l'une des revendications 1 à 6,
**caractérisé en ce**
**que** le médiateur est une 1,10-phénanthroline-5,6-quinone de formule générale (I), une 1,7-phénanthroline-5,6-quinine de formule générale (II) ou une 4,7-phénanthroline-5,6-quinone de formule générale (III), 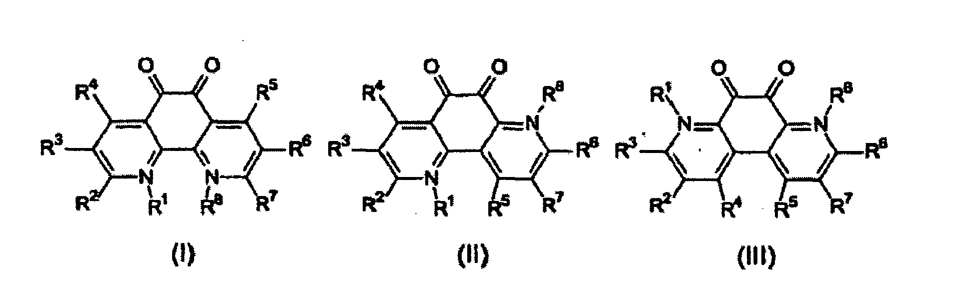 dans laquelle
R² à R⁷ sont indépendamment l'un de l'autre, un atome d'hydrogène, d'halogène, OH, un groupe O(alkyle), OCO(alkyle), S(alkyle), NH₂, NH(alkyle), N(alkyle)₂, [N(alkyle)₃]⁺, CN, NO₂, COOH, SO₃H ou un radical alkyle linéaire ou ramifié, un radical cycloalkyle, un radical aryle ou un radical hétéroaryle qui peuvent respectivement être éventuellement mono- ou poly-substitués, et
R¹ et R⁸ sont indépendamment l'un de l'autre une paire d'électrons libres, H ou un radical alkyle linéaire ou ramifié, qui peut éventuellement être mono- ou poly-substitué,
ou un sel de ceux-ci.

8. Procédé selon l'une des revendications 1 à 7,
**caractérisé en ce que** le médiateur est la N-méthyl-1,10-phénanthrolinium-5,6-quinone, la N,N'-diméthyl-1,10-phénanthrolinium,5,6-quinone, la N-méthyl-1,7-phénanthrolinium-5,6-quinone, la N,N'-diméthyl-1,7-phénanthrolinium-5,6-quinone, la N-méthyl-4,7-phénanthrolinium-5,6-quinone ou la N,N'-diméthyl-4,7-phénanthrolinium-5,6-quinone.

9. Procédé selon l'une des revendications 1 à 8,
**caractérisé en ce**
**que** l'indicateur optique est l'acide molybdatophosphorique.

10. Procédé selon l'une des revendications 1 à 9,
**caractérisé en ce**
**que** le procédé est réalisé sur un support, le support comprenant :
(a) une zone d'application pour appliquer l'échantillon,
(b) une zone réactionnelle pour convertir l'analyte avec le réactif de détection,
(c) une zone de détection pour déterminer la présence ou/et la quantité d'analyte dans l'échantillon par une détection optique de l'indicateur ou du système d'indicateur, et
(d) éventuellement, une zone de déchet.

11. Procédé selon la revendication 10,
**caractérisé en ce**
**que** l'échantillon est appliqué directement sur la zone d'application.

12. Procédé selon la revendication 10,
**caractérisé en ce**
**que** l'échantillon est absorbé au moyen d'un élément de transfert et ensuite, est appliqué, éventuellement après humidification, sur la zone d'application.

13. Procédé selon l'une quelconque des revendications 10 à 12,
**caractérisé en ce**
**que** le support est un élément de test.

14. Réactif de détection optique d'un analyte dans un échantillon, comprenant
(a) une oxydoréductase dépendante du nicotinamide,
(b) une co-enzyme de nicotinamide réductible,
(c) un médiateur qui est une quinone, et
(d) un indicateur optique réductible ou un système d'indicateur optique réductible, dans lequel l'oxydoréducrase dépendante du nicotinamide est l'alcool-déshydrogénase (EC 1.1.1.1), la glucose déshydrogénase (EC 1.1.1.47) ou la glucose-6-phosphate-déshydrogénase (EC 1.1.1.49), l'indicateur optique est un hétéropolyacide et le médiateur est une 1,10-phénanthroline-quinone, une 1,7-phénanthroline-quinone, une 4,7-phénanthroline-quinone ou un sel N-alkylé ou N,N'-dialkylé de ceux-ci.

15. Utilisation d'un réactif de détection selon la revendication 14, dans un élément de test.

16. Kit comprenant un réactif de détection selon la revendication 14, et un élément de test, pour la détection optique d'un analyte dans un échantillon, l'élément de test comprenant :
(a) une zone d'application pour appliquer l'échantillon,
(b) une zone réactionnelle pour convertir l'analyte avec le réactif de détection,
(c) une zone de détection pour déterminer la présence ou/et la quantité d'analyte dans l'échantillon par une détection optique de l'indicateur ou du système d'indicateur, et
(d) éventuellement, une zone de déchet.

17. Elément de test pour la détermination optique d'un analyte dans un échantillon, comprenant
(a) une zone d'application pour appliquer l'échantillon,
(b) une zone réactionnelle qui contient un réactif de détection, comprenant une oxydoréductase dépendante du nicotinamide, une co-enzyme de nicotinamide réductible, un médiateur qui est une quinone et un indicateur optique réductible ou un système d'indicateur optique réductible, pour convertir l'analyte avec le réactif de détection,
(c) une zone de détection pour déterminer la présence ou/et la quantité d'analyte dans l'échantillon par une détection optique de l'indicateur ou du système d'indicateur, et
(d) éventuellement, une zone de déchet,
dans lequel l'oxydoréductase dépendante du nicotinamide est l'alcool-déshydrogénase (EC 1.1.1.1), la glucose déshydrogénase (EC 1.1.1.47) ou la glucose-6-phosphate-déshydrogénase (EC 1.1.1.49), l'indicateur optique est un hétéropolyacide et le médiateur est une 1,10-phénanthroline-quinone, une 1,7-phénanthroline-quinone, une 4,7-phénanthroline-quinone ou un sel N-alkylé ou N,N'-dialkylé de ceux-ci.
